# EUROPÄISCHE PATENTANMELDUNG

(11) **EP 4 523 653 A1**
(43) Veröffentlichungstag der Anmeldung: **19.03.2025**
(21) Anmeldenummer: 24188191.1
(22) Anmeldetag: 12.07.2024
(51) Int. Cl.: A61F 2/00, A61F 2/12

(54) **MEDIZINISCHES IMPLANTAT ZUR AUFFÜLLUNG EINES HOHLRAUMS IN EINEM DRÜSENGEWEBE UND VERFAHREN ZUR HERSTELLUNG EINES MEDIZINISCHEN IMPLANTATS ZUR AUFFÜLLUNG EINES HOHLRAUMS IN EINEM DRÜSENGEWEBE**

(30) Priorität: 12.09.2023 IT 202300018666
(71) Anmelder: pfm medical gmbh, 50996 Köln (DE)
(72) Erfinder: Rieck, Monika, 50996 Köln (DE)
(74) Vertreter: Lippert Stachow Patentanwälte Rechtsanwälte Part.mbB

(57) **Zusammenfassung**

Die Erfindung betrifft ein medizinisches Implantat (1) zur Auffüllung eines Hohlraums in einem Drüsengewebe, insbesondere im Drüsengewebe der menschlichen Brust, wobei das medizinische Implantat (1) aus einem titanisierten Polymer besteht und als Netz ausgebildet ist, um den aufzufüllenden Hohlraum zu bedecken. Die Erfindung betrifft ferner ein Verfahren zu Herstellung des medizinischen Implantats (1).

## Beschreibung

Die Erfindung betrifft ein medizinisches Implantat zur Auffüllung eines Hohlraums in einem Drüsengewebe, insbesondere im Drüsengewebe der menschlichen Brust. Die Erfindung betrifft ferner ein Verfahren zur Herstellung eines medizinischen Implantats zur Auffüllung eines Hohlraums in einem Drüsengewebe.

Das erfindungsgemäße Implantat dient insbesondere zur Auffüllung eines Hohlraums in der menschlichen Brust nach einer brusterhaltenden Brustkrebs-Operation. Bei der brusterhaltenden Operation entfernt der Chirurg das Tumorgewebe vollständig aus dem Brustgewebe (Drüsengewebe). Der Tumor wird dabei mit einem möglichst kleinen Rand des umliegenden gesunden Gewebes entfernt. Die Randbereiche (Schnittränder) des entfernten Gewebes und/oder das Tumorbett (Bereich der Brust, wo der Tumor lag) werden nach der Operation im Labor auf Krebszellen untersucht. Neuere Methoden erlauben auch eine Untersuchung der Randbereiche des entfernten Gewebes während der Operation. Wenn sich bei der feingeweblichen Untersuchung herausstellt, dass das Krebsgewebe nicht komplett entfernt wurde, muss während der Operation nachgeschnitten werden bzw. eine neue Operation zum Nachschneiden durchgeführt werden.

Durch das Entfernen des Tumors aus dem Brustgewebe entsteht jedoch ein Hohlraum, welcher von außen sichtbar sein kann. Vorzugsweise wird dieser Hohlraum aufgefüllt, damit dieser nicht von außen sichtbar ist. Allerdings führen insbesondere kleine eingesetzte Implantate häufig zu Verkapselungen, Entzündungen, Wucherungen oder gar abstoßenden Reaktionen.

Eine Alternative zu der brusterhaltenden Operation von Brustkrebs ist die vollständige Entfernung der Brust (Mastektomie). Die entfernte Brust kann nachfolgend wieder aufgebaut werden (Brustrekonstruktion). Dazu offenbart beispielsweise die WO2013/148719 A1 ein Netzimplantat zur Aufnahme eines Brustimplantats. Das Netzimplantat wird am Brustmuskel fixiert, so dass das darin aufgenommene Implantat nicht verrutschen kann. Zur Verbesserung der Biokompatibilität ist das Netzimplantat vorzugsweise beschichtet, insbesondere titanisiert.

Ausgehend von diesem Stand der Technik liegt der Erfindung die Aufgabe zugrunde, ein medizinisches Implantat zur Auffüllung eines Hohlraums in einem Drüsengewebe bereitzustellen und gleichzeitig Verkapselungen, Entzündungen, Wucherungen oder abstoßende Reaktionen zu verhindern.

Die Aufgabe wird erfindungsgemäß gelöst durch ein medizinisches Implantat zur Auffüllung eines Hohlraums in einem Drüsengewebe, insbesondere im Drüsengewebe der menschlichen Brust, wobei das medizinische Implantat aus einem titanisierten Polymer besteht und als Netz ausgebildet ist, um den aufzufüllenden Hohlraum zu bedecken.

Die Netzstruktur des erfindungsgemäßen medizinischen Implantats sorgt dafür, dass das umliegende Drüsengewebe (Brustgewebe) in die Netzstruktur einwachsen kann. Gleichzeitig verhindert die Verwendung eines titanisierten Polymers das Entstehen von Verkapselungen, Entzündungen, Wucherungen oder abstoßende Reaktionen. Das erfindungsgemäße medizinische Implantat erzeugt also ein Wachstum des umliegenden Gewebes, insbesondere des umliegenden Tumorbetts, in die Netzstruktur des Implantats, wodurch sich der Hohlraum durch Eigengewebe füllt. Durch die Biokompatibilität des titanisierten Polymers enthält dieses neugewachsene Gewebe keine Verkapselungen oder Wucherungen, welche insbesondere durch entzündliche Prozesse oder Abstoßreaktionen entstehen.

Ein titanisiertes Polymer im Sinne der Erfindung ist eine Polymerfaser, welche mit Titan beschichtet ist. Zweckmäßigerweise ist die Titanbeschichtung so dünn aufgebracht, dass die Elastizität und Flexibilität der Polymerfaser nicht eingeschränkt ist. Vorzugweise ist das medizinische Implantat aus einem Polymernetz hergestellt, welches nachfolgend titanisiert wird. Dies hat den Vorteil, dass die nachfolgend aufgebrachte Titanschicht die Netzstruktur fixiert, so dass das medizinische Implantat zu einem späteren Zeitpunkt zugeschnitten werden kann, ohne dass sich die Netzstruktur auflöst.

Nach einer zweckmäßigen Variante der Erfindung besteht das medizinische Implantat aus einem titanisierten Polypropylen.

In einer vorteilhaften Variante der Erfindung weist das medizinische Implantat die Form eines Pluszeichens (+) auf. Der Schnittpunkt der beiden Schenkel des Pluszeichens kann an der tiefsten Stelle des aufzufüllenden Hohlraums positioniert werden, so dass die sich davon erstreckenden Schenkel die Seitenwände des aufzufüllenden Hohlraums zumindest teilweise, vorzugsweise vollständig, bedecken. Ragen Teile der Schenkel aus dem aufzufüllenden Hohlraum heraus, können diese nach dem erfolgten Einsetzen des erfindungsgemäßen medizinischen Implantats abgeschnitten werden. In dieser Form lässt sich das erfindungsgemäße Implantat besonders einfach an die Größe, insbesondere Tiefe, des aufzufüllenden Hohlraums anpassen.

Gemäß einer erfindungsgemäßen Variante weist das Netz eine Maschenweite kleiner 2 mm auf, vorzugsweise eine Maschenweite von 1 mm. Diese Maschenweite ist besonders vorteilhaft für das Einwachsen des umliegenden Gewebes, insbesondere des Drüsengewebes bzw. Brustgewebes.

Nach einer zweckmäßigen Variante der Erfindung weist das medizinische Implantat ein Gewicht von weniger als 50 g/m² auf, insbesondere von 16 g/m² oder 35 g/m².

In einer besonders zweckmäßigen Variante der Erfindung ist die Dicke der Titanschicht so gewählt, dass die Elastizität und Flexibilität des Polymernetzes nicht beeinträchtigt wird. Beispielsweise weist die Polymerfaser des Netzes einen Durchmesser von 50 bis 100 µm auf und die Titanbeschichtung eine Dicke im Bereich von 15 bis 75 nm.

Die Aufgabe wird ferner gelöst durch ein Verfahren zur Herstellung eines erfindungsgemäßen medizinischen Implantats umfassend die Schritte:
Bereitstellen eines Netzes aus einem titanisierten Polymer, wobei die Abmessungen des Netzes größer sind als das herzustellende medizinische Implantat,
Ausschneiden von Bereichen des bereitgestellten Netzes, um die gewünschte Form des medizinischen Implantats zu erhalten, wobei die erhaltene Form des gewünschten Implantats an vordefinierten Stellen mit dem verbleibenden Gewebe des bereitgestellten Netzes verbunden bleibt.

Das erfindungsgemäße medizinische Implantat ist somit bis kurz vor der tatsächlichen Implantation ein Teil des bereitgestellten Netzes mit größeren Abmessungen als das tatsächliche medizinische Implantat. Erst unmittelbar vor der Implantation werden die vordefinierten Verbindungsstellen getrennt, wodurch das medizinische Implantat aus der umgebenden Netzstruktur gelöst wird. Dadurch verbessert sich die Handhabbarkeit des medizinischen Implantats bis zur tatsächlichen Implantation.

In einer bevorzugten Variante des erfindungsgemäßen Verfahrens werden wenigstens zwei medizinische Implantate unterschiedlicher Größe in dem bereitgestellten Netz ausgeschnitten. Es werden also in der Netzstruktur mehrere medizinische Implantate unterschiedlicher Größe bereitgestellt und der Chirurg kann während der Operation das medizinische Implantat aus dem Netz herauslösen, welche der Größe des aufzufüllenden Hohlraums entspricht. Wie bereits einleitend ausgeführt, wird das Tumorgewebe zusammen mit einem Rand des umliegenden Gewebes entfernt. Es lässt sich vorab nicht genau vorhersagen, wie groß der aufzufüllende Hohlraum sein wird, insbesondere wenn während der Operation nachgeschnitten werden muss, weil z.B. eine Feingewebeuntersuchung während der Operation ergeben hat, dass nicht das gesamte Tumorgewebe entfernt wurde. Dadurch, dass aus dem Netz mehrere medizinische Implantate unterschiedlicher Größe ausgeschnitten wurden, kann der Chirurg durch Trennen der definierten Verbindungsstellen das medizinische Implantat auswählen, welches die ideale Größe für den aufzufüllenden Hohlraum aufweist. Ferner kann der Chirurg den Sitz der unterschiedlichen medizinischen Implantate in dem aufzufüllenden Hohlraum austesten, bevor er sich für ein medizinisches Implantat entscheidet. Das Bereitstellen von mehreren medizinischen Implantaten unterschiedlicher Größe in einem Netz ist dabei wirtschaftlicher als das Bereitstellen separater Netze mit jeweils einem medizinischen Implantat, da diese einzeln verpackt, sterilisiert, gelagert, usw. werden müssten.

Gemäß einer zweckmäßigen Variante der Erfindung umfasst das Verfahren den Schritt des Verpackens und Sterilisierens des bereitgestellten Netzes und des wenigstens einen darin ausgeschnittenen medizinischen Implantats.

Nach einer vorteilhaften Variante der Erfindung wird das titanisierte Netz hergestellt durch:
Bereitstellen eines Polymernetzes, und
Aufbringen einer Titanschicht auf das bereitgestellte Polymernetz.

Durch das nachfolgende Aufbringen der Titanschicht wird die Netzstruktur besser fixiert, so dass ein Schneiden des Netzes keinen negativen Einfluss auf die Netzstruktur hat.

In einer besonders zweckmäßigen Variante der Erfindung ist die Dicke der Titanschicht so gewählt, dass die Elastizität und Flexibilität des Polymernetzes nicht beeinträchtigt wird. Beispielsweise weist die Polymerfaser des Netzes einen Durchmesser von 50 bis 100 µm auf und die Titanbeschichtung eine Dicke im Bereich von 15 bis 75 nm.

Nachfolgend wird die Erfindung anhand von in den Figuren dargestellten Ausführungsbeispielen näher erläutert. Es zeigen:
- Fig. 1: eine schematische Ansicht eines erfindungsgemäßen medizinischen Implantats,
- Fig. 2: eine schematische Ansicht eines titanisierten Polymernetzes mit zwei darin ausgeschnittenen erfindungsgemäßen medizinischen Implantaten, und
- Fig. 3: ein Ablaufdiagram eines erfindungsgemäßen Verfahrens zur Herstellung eines erfindungsgemäßen medizinischen Implantats.

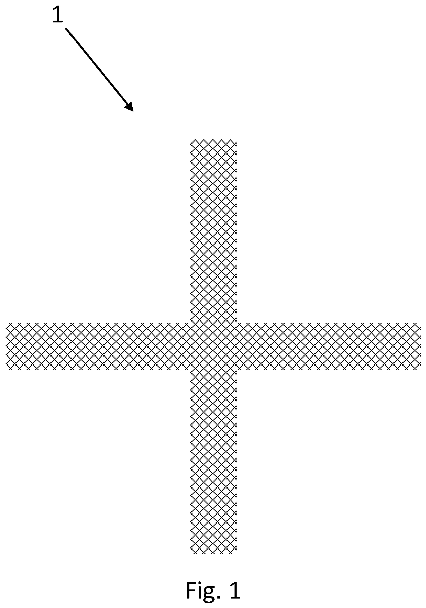

Fig. 1 zeigt eine schematische Ansicht eines erfindungsgemäßen medizinischen Implantats 1 zur Auffüllung eines Hohlraums in einem Drüsengewebe, insbesondere im Drüsengewebe der menschlichen Brust. Das medizinische Implantat 1 aus Fig. 1 besteht aus einem titanisierten Polymer und ist als Netz ausgebildet, um den aufzufüllenden Hohlraum zu bedecken.

Dadurch, dass das erfindungsgemäße medizinische Implantat 1 als Netz ausgebildet ist und im implantierten Zustand den aufzufüllenden Hohlraum bedeckt, kann das umliegende Drüsengewebe (Brustgewebe) in die Netzstruktur des medizinischen Implantats 1 einwachsen. Gleichzeitig verhindert die Verwendung eines titanisierten Polymers das Entstehen von Verkapselungen, Entzündungen, Wucherungen oder abstoßende Reaktionen. Das erfindungsgemäße medizinische Implantat 1 fördert ein Wachstum des umliegenden Gewebes, welches von dem medizinischen Implantat 1 im implantierten Zustand bedeckt wird, in die Netzstruktur des medizinischen Implantats 1. Durch das erzeugte Wachstum des umliegenden Gewebes füllt sich der Hohlraum durch Eigengewebe. Die Biokompatibilität des titanisierten Polymers verhindert Verkapselungen oder Wucherungen in dem neugewachsenen Gewebe, welche insbesondere durch entzündliche Prozesse oder Abstoßreaktionen entstehen.

Ein titanisiertes Polymer im Sinne der Erfindung ist eine Polymerfaser, welche mit Titan beschichtet ist. Zweckmäßigerweise ist die Titanbeschichtung so dünn aufgebracht, dass die Elastizität und Flexibilität der Polymerfaser nicht eingeschränkt ist. Vorzugweise ist das medizinische Implantat 1 aus einem Polymernetz hergestellt, welches nachfolgend titanisiert wird. Dies hat den Vorteil, dass die nachfolgend aufgebrachte Titanschicht die Netzstruktur fixiert, so dass das medizinische Implantat 1 zu einem späteren Zeitpunkt zugeschnitten werden kann, ohne dass sich die Netzstruktur auflöst.

Vorzugweise besteht das medizinische Implantat 1 aus einem titanisierten Polypropylen. Die Netzstruktur des medizinischen Implantats 1 weist bevorzugt eine Maschenweite kleiner 2 mm, insbesondere eine Maschenweite von ungefähr 1 mm. Zweckmäßigerweise weist das medizinische Implantat 1 ein Gewicht von weniger als 50 g/m² auf, insbesondere von 16 g/m² oder 35 g/m².

Wie in der Fig. 1 dargestellt, weist das medizinische Implantat 1 die Form eines Pluszeichens auf, bzw. eine X-Form nach einer Drehung um 45°. Der Schnittpunkt der beiden Schenkel des Pluszeichens wird bei der Implantation des medizinischen Implantats 1 an der tiefsten Stelle des aufzufüllenden Hohlraums positioniert und die sich davon erstreckenden Schenkel bedecken die Seitenwände des aufzufüllenden Hohlraums zumindest teilweise, vorzugsweise vollständig. Um das erfindungsgemäße Implantat besonders einfach an die Größe, insbesondere Tiefe, des aufzufüllenden Hohlraums anzupassen, können die Teile der Schenkel, die aus dem aufzufüllenden Hohlraum herausragen, abgeschnitten werden.

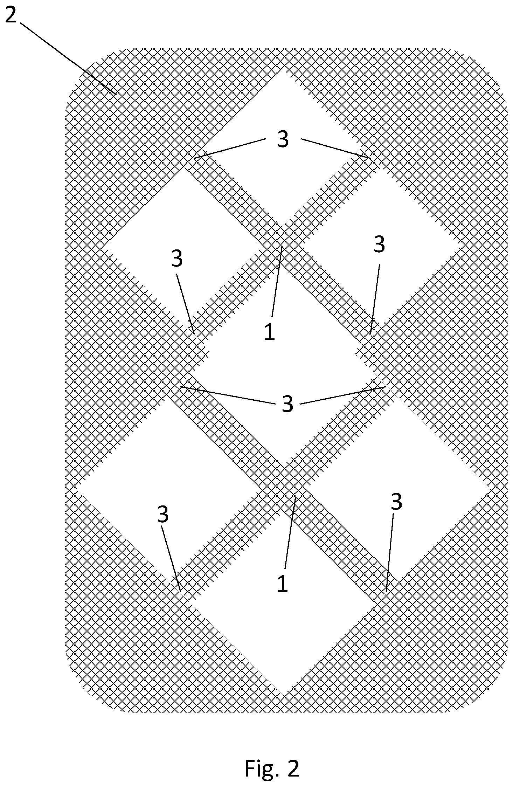

Fig. 2 zeigt eine schematische Ansicht eines titanisierten Polymernetzes 2 mit zwei darin ausgeschnittenen erfindungsgemäßen medizinischen Implantaten 1. Die beiden medizinischen Implantate 1 sind mit dem Netz 2 an vordefinierten Stellen 3 verbunden und können vor der Implantation aus dem Netz 2 gelöst (geschnitten) werden.

Die beiden in dem Netz 2 ausgeschnittenen medizinischen Implantate 1 weisen unterschiedliche Größen auf, so dass der Chirurg während der Operation das medizinische Implantat 1 in der passenden Größe aussuchen kann. Auch kann der Chirurg den Sitz der beiden medizinischen Implantate 1 miteinander vergleichen und auf dieser Basis die passende Größe des medizinischen Implantats 1 bestimmen.

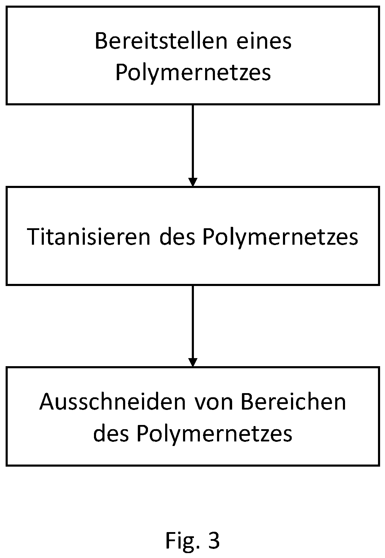

Fig. 3 zeigt ein Ablaufdiagram eines erfindungsgemäßen Verfahrens zur Herstellung eines erfindungsgemäßen medizinischen Implantats 1. In einem ersten Schritt wird ein Netz 2 aus Polymerfasern bereitgestellt. Beispielsweise haben die Polymerfasern einen Durchmesser von 50 bis 100 µm. Die Abmessungen des Netzes 2 sind größer als das herzustellende medizinische Implantat 1 bzw. die daraus herzustellenden medizinischen Implantate 1.

Im nächsten Schritt wird auf das bereitgestellte Netz eine Titanschicht aufgebraucht. Die Titanschicht weist beispielsweise eine Dicke im Bereich von 15 bis 75 nm auf. Zweckmäßigerweise beeinträchtigt die aufgebrachte Titanschicht nicht die Elastizität und Flexibilität des Polymernetzes 2.

Im letzten Schritt des Herstellungsverfahrens werden Bereiche des bereitgestellten und titanisierten Netzes 2 ausgeschnitten, um die gewünschte Form des medizinischen Implantats 1 zu erhalten. Wie in Fig. 2 dargestellt, können aus dem Netz 2 auch mehrere medizinische Implantate 1 unterschiedlicher Größe ausgeschnitten werden.

Die erhaltene Form des gewünschten Implantats 1 bleibt an vordefinierten Stellen 3 mit dem verbleibenden Gewebe des bereitgestellten Netzes 2 verbunden.

Abschließend kann das bereitgestellte Netz 2 inklusive des darin ausgeschnittenen medizinischen Implantats 1 verpackt und sterilisiert werden. In diesem Zustand wird das verpackte Netz 2 transportiert und gelagert, bis es durch einen Chirurgen in einer brusterhaltenen Operation in den aufzufüllenden Hohlraum eingesetzt wird. Während der Operation kann der Chirurg das medizinische Implantat 1 aus dem umgebenden Netz 2 lösen, in dem die vordefinierten Stellen getrennt, insbesondere durchschnitten, werden.

### Bezugszeichenliste

- 1: Medizinisches Implantat
- 2: Netz
- 3: vordefinierte Verbindungsstelle

## Patentansprüche

1. Medizinisches Implantat (1) zur Auffüllung eines Hohlraums in einem Drüsengewebe, insbesondere im Drüsengewebe der menschlichen Brust,
wobei das medizinische Implantat (1) aus einem titanisierten Polymer besteht und als Netz ausgebildet ist, um den aufzufüllenden Hohlraum zu bedecken.

2. Medizinisches Implantat (1) nach Anspruch 1, wobei das medizinische Implantat (1) aus einem titanisierten Polypropylen besteht.

3. Medizinisches Implantat (1) nach Anspruch 1 oder Anspruch 2, wobei das medizinische Implantat (1) die Form eines Pluszeichens aufweist.

4. Medizinisches Implantat (1) nach einem der Ansprüche 1 bis 3, wobei das Netz eine Maschenweite kleiner 2 mm aufweist, vorzugsweise eine Maschenweite von 1 mm.

5. Medizinisches Implantat (1) nach einem der Ansprüche 1 bis 4, wobei das medizinische Implantat (1) ein Gewicht von weniger als 50 g/m² aufweist, insbesondere von 16 g/m² oder 35 g/m².

6. Verfahren zur Herstellung eines medizinisches Implantats (1) nach einem der Ansprüche 1 bis 5, umfassend die Schritte:
Bereitstellen eines Netzes (2) aus einem titanisierten Polymer, wobei die Abmessungen des Netzes (2) größer sind als das herzustellende medizinische Implantat (1),
Ausschneiden von Bereichen des bereitgestellten Netzes (2), um die gewünschte Form des medizinischen Implantats (1) zu erhalten, wobei die erhaltene Form des gewünschten Implantats (1) an vordefinierten Stellen (3) mit dem verbleibenden Gewebe des bereitgestellten Netzes (2) verbunden bleibt.

7. Verfahren nach Anspruch 6, wobei wenigstens zwei medizinische Implantate (1) unterschiedlicher Größe in dem bereitgestellten Netz (2) ausgeschnitten werden.

8. Verfahren nach Anspruch 6 oder Anspruch 7, umfassend den Schritt des Verpackens und Sterilisierens des bereitgestellten Netzes (2) und des wenigstens einen darin ausgeschnittenen medizinischen Implantats (1).

9. Verfahren nach einem der Ansprüche 6 bis 8, wobei das titanisierte Netz (2) hergestellt wird durch:
Bereitstellen eines Polymernetzes, und
Aufbringen einer Titanschicht auf das bereitgestellte Polymernetz.

10. Verfahren nach Anspruch 9, wobei die Dicke der Titanschicht so gewählt ist, dass die Elastizität und Flexibilität des Polymernetzes nicht beeinträchtigt wird.
